# EUROPEAN PATENT APPLICATION

(11) **EP 4 678 165 A1**
(43) Date of publication of application: **14.01.2026**
(21) Application number: 24766260.4
(22) Date of filing: 22.02.2024
(51) Int. Cl.: A61K 9/16, A61K 31/437, A61K 47/34, A61P 25/18

(54) **LONG-ACTING AND SUSTAINED-RELEASE PREPARATION COMPOSITION OF LUMATEPERONE AND PREPARATION METHOD THEREFOR**

(30) Priority: 03.03.2023 CN 202310203435
(71) Applicant: Sichuan Kelun Pharmaceutical Research Institute Co., Ltd., Chengdu, Sichuan 611138 (CN); Hunan Kelun Pharmaceutical Research Co., Ltd., Yueyang, Hunan 414000 (CN)
(72) Inventor: SU, Zhengxing, Chengdu, Sichuan 611138 (CN); DING, Duohao, Chengdu, Sichuan 611138 (CN); ZHOU, Miaomiao, Chengdu, Sichuan 611138 (CN); BAO, Fei, Chengdu, Sichuan 611138 (CN); ZHANG, Xiaohang, Chengdu, Sichuan 611138 (CN); ZHAO, Dong, Chengdu, Sichuan 611138 (CN); LIU, Sichuan, Chengdu, Sichuan 611138 (CN)
(74) Representative: Hoffmann Eitle
(86) International application number: PCT/CN2024/078008
(87) International publication number: WO 2024/183538

(57) **Abstract**

Disclosed in the present application is a long-acting and sustained-release preparation composition of lumateperone, the composition comprising lumateperone or a salt thereof and a polymer material, wherein the mass ratio of lumateperone to the polymer material is 0.5 : 9.5 to 5 : 5; the polymer material comprises a main material, i.e. polylactide, and an optional release regulator; the mass of the release regulator is 0% to 95% of the mass of the main material, i.e. polylactide; and the release regulator is selected from one of or a mixture of more than one of polylactide or polylactide-glycolide. The long-acting and sustained-release preparation of the present invention can achieve a sustained-release effect ranging from one week to three months, can improve the compliance of a patient, and has no obvious burst release in the early stage and no release lag phase, has stable release of the preparation, and ensures the safety and effectiveness of drug release.

## Description

### Technical Field

The present invention relates to a lumateperone long-acting sustained-release preparation composition and a preparation method thereof.

### Background Art

Schizophrenia is a common chronic and highly disabling mental disorder affecting approximately 1% of the global population. Antipsychotic therapy is the main approach for treating schizophrenia. However, currently the remission achieved by drug treatment is limited, and various adverse reactions often occur during the treatment. In December 2019, the U.S. Food and Drug Administration approved a new antipsychotic drug, lumateperone. This drug exhibits a unique mechanism of action, demonstrates superior safety and tolerability profiles, particularly in alleviating positive symptoms while improving negative symptoms, depressive symptoms, and social functioning in patients with schizophrenia.

Lumateperone, a novel small-molecule antipsychotic drug, was first successfully developed by Bristol-Myers Squibb Company and was exclusively licensed to Intra-Cellular Therapies Company in 2005. Lumateperone is a first in class drug that selectively regulates serotonin, dopamine (DA) and glutamate simultaneously. In December 2019, this drug was approved for marketing by FDA in the United States for the treatment of schizophrenia in adults. In addition to its application in the treatment of schizophrenia, lumateperone also has potential therapeutic effects on bipolar depression, behavioral disturbances associated with dementia and Alzheimer's disease, insomnia, and major depressive disorder (MDD). At the same time, it also has better tolerability, and is less likely to cause adverse reactions such as weight gain and abnormal lipid metabolism.

At present, lumateperone tosylate capsules have been marketed. This product is an oral preparation that needs to be administered daily to maintain its plasma drug concentration. The need for frequent administration leads to poor medication compliance among patients.

CN115671057A discloses a lumateperone long-acting microsphere sustained-release preparation, in which a poly(lactide-co-glycolide) (PLGA) polymer material is used to prepare microspheres. However, the microspheres have the following problems: i) the particle size distribution is relatively narrow, resulting in an overly concentrated release behavior of the microspheres, which is not conducive to maintaining a stable plasma drug concentration during the administration period; ii) there is a relatively long lag time in the body, and the release is not steady; and iii) the burst release at the early stage is too high.

### Summary of the Invention

According to a first aspect of the present invention, there is provided a lumateperone long-acting sustained-release preparation composition comprising lumateperone or a salt thereof and a polymer material, wherein the mass ratio of lumateperone to the polymer material is 0.5:9.5 to 5:5, the polymer material comprises a main material polylactide (PLA) and optionally a release regulator, the mass of the release regulator is 0% to 95% of the mass of the main material polylactide, and the release regulator is selected from one of or a mixture of more than one of polylactide or poly(lactide-co-glycolide).

In some embodiments of the present invention, the mass ratio of lumateperone to the polymer material is 1:9 to 4:6; preferably, the mass ratio of lumateperone to the polymer material is 1.5:8.5 to 3:7; preferably, the mass ratio of lumateperone to the polymer material is 2:8 to 3:7; more preferably, the mass ratio of lumateperone to the polymer material is 2:(6-8); more preferably, the mass ratio of lumateperone to the polymer material is 2:(6.5-7).

In some embodiments of the present invention, the main material polylactide has a molecular weight of 5,000 to 100,000 Daltons, preferably 10,000 to 80,000 Daltons, more preferably 10,000 to 50,000 Daltons, more preferably 20,000 to 30,000 Daltons, more preferably 25,000 Daltons.

In some embodiments of the present invention, the mass of the release regulator is 0% to 50%; preferably 0% to 40%; more preferably 0% to 35%; more preferably 0% to 30%, of that of the main material polylactide.

In some embodiments of the present invention, the poly(lactide-co-glycolide) in the release regulator has a molar ratio of lactide to glycolide of 50:50 to 95:5, and the poly(lactide-co-glycolide) has a molecular weight of 5,000 to 100,000 Daltons; preferably, the molar ratio of lactide to glycolide is 50:50 to 85:15, and the poly(lactide-co-glycolide) has a molecular weight of 5,000 to 90,000 Daltons; more preferably, the molar ratio of lactide to glycolide is 50:50 to 75:25, and the poly(lactide-co-glycolide) has a molecular weight of 10,000 to 80,000 Daltons; more preferably, the molar ratio of lactide to glycolide is 50:50 to 75:25, and the poly(lactide-co-glycolide) has a molecular weight of 60,000 to 70,000 Daltons.

In some embodiments of the present invention, the polylactide in the release regulator has a molecular weight of 5,000 to 100,000 Daltons, preferably 5,000 to 80,000 Daltons, more preferably 5,000 to 50,000 Daltons.

In some embodiments of the present invention, the release regulator is poly(lactide-co-glycolide) (PLGA) having a molar ratio of lactide to glycolide of 50:50 to 85:15 and a molecular weight of 5,000 to 90,000 Daltons; preferably, the release regulator is poly(lactide-co-glycolide) (PLGA) having a molar ratio of lactide to glycolide of 50:50 to 75:25 and a molecular weight of 10,000 to 80,000 Daltons; preferably, the release regulator is poly(lactide-co-glycolide) having a molar ratio of lactide to glycolide of 75:25 and a molecular weight of 10,000 to 80,000 Daltons; preferably, the release regulator is poly(lactide-co-glycolide) having a molar ratio of lactide to glycolide of 75:25 and a molecular weight of 60,000 to 70,000 Daltons.

In some embodiments of the present invention, the polymer material is polylactide.

In some embodiments of the present invention, the polymer material is a mixture of polylactide and poly(lactide-co-glycolide), and the mass of poly(lactide-co-glycolide) is 20% to 50% of that of polylactide.

In some embodiments of the present invention, the salt of lumateperone is selected from a group consisting of erucate, oleate, α-linolenate, palmitate, heptadecanoicate, undecylenate, hexadecanedionate, laurate, stearate, adipate and benzoate of lumateperone.

In some embodiments of the present invention, the lumateperone long-acting sustained-release preparation is selected from microspheres or subcutaneous implants.

According to a second aspect of the present invention, there is provided a method for preparing the lumateperone long-acting sustained-release microspheres, comprising the steps of:
dissolving the polymer material and the lumateperone or a salt thereof in a solvent, sterilizing by filtration to obtain an oil phase, and controlling the temperature at 2-35°C; preparing a 0.2% to 5% polyvinyl alcohol (PVA) aqueous solution, sterilizing by filtration to obtain an aqueous phase, and controlling the temperature at 2-35°C; adding the oil phase into the aqueous phase in a shearing machine to shear to form a primary emulsion, then volatilizing under stirring, filtering, washing microspheres with distilled water, and lyophilizing to obtain the lumateperone long-acting sustained-release microspheres.

In some embodiments of the method of the present invention, the polymer material and lumateperone or a salt thereof are dissolved in dichloromethane.

In some embodiments of the method of the present invention, the rotational speed of the shearing machine is 1,000-8,000 rpm.

In some embodiments of the method of the present invention, the primary emulsion is volatilized under stirring at 50-500 rpm, preferably the primary emulsion is volatilized under stirring at 100-200 rpm.

According to a third aspect of the present invention, there is provided a method for preparing the lumateperone long-acting sustained-release subcutaneous implants, comprising the steps of:
mixing the polymer material and the lumateperone or a salt thereof uniformly, extruding, then drawing into filaments and cutting into pellets, and sterilizing by irradiation to obtain the lumateperone long-acting sustained-release subcutaneous implants.

In some embodiments of the method of the present invention, extrusion is perfomed with a twin-screw extruder.

In some embodiments of the method of the present invention, the extrusion parameters of the twin-screw extruder are as follows: feeding temperature: 15-30°C, conveying temperature: 35-40°C, kneading temperature: 60-100°C, degassing temperature: 80-100°C, extrusion temperature: 50-80°C, die temperature: 50-60°C, extrusion speed: 80-120 rpm, torque: 7-8 N.cm.

In some embodiments of the method of the present invention, the extrusion parameters of the twin-screw extruder are as follows: feeding temperature: room temperature, conveying temperature: 35-40°C, kneading temperature: 80-100°C, degassing temperature: 100°C, extrusion temperature: 80°C, die temperature: 60°C, extrusion speed: 100 rpm, torque: 7-8 N.cm.

In the context of the present application, the term "molecular weight" refers to the weight average molecular weight of a polymer.

The long-acting sustained-release preparation of the present invention can achieve a sustained-release effect varying from 1 week to 3 months, and can improve the compliance of patients. Moreover, the preparation has no significant burst release at the early stage and no release lag time, and the preparation has a steady release, ensuring the safety and effectiveness of drug release.

In the present invention, the microspheres are prepared by using an emulsification technology, which can well control the particle size distribution, thereby obtaining a pharmacokinetic curve with a steady release.

### Brief Description of the Drawings

FIG. 1 is pharmacokinetic profiles of the microspheres of Examples 1, 2, and 3 at day 0 to day 20 (25 or 60).
FIG. 2 is pharmacokinetic profiles of the microspheres of Examples 1, 2, and 3 at day 0 to day 1.
FIG. 3 is a pharmacokinetic profile of the microspheres of Example 2 at day 0 to day 25.
FIG. 4 is a pharmacokinetic profile of the microspheres of Example 2 at day 0 to day 1.
FIG. 5 is pharmacokinetic profiles of the microspheres of Example 3 and the subcutaneous implants of Example 4 at day 0 to day 60.
FIG. 6 is pharmacokinetic profiles of the microspheres of Examples 3 and 5 at day 0 to day 60 (100).
FIG. 7 is pharmacokinetic profiles of the microspheres of Examples 3 and 5 at day 0 to day 1.

### Examples

The present invention will be further illustrated by the following examples. The examples are only used to illustrate the technical solution of the present invention, and are not intended to limit the scope of the present invention. Those skilled in the art can make some non-essential improvements and adjustments, which remain within the scope of the present invention.

Unless otherwise specified, each step in the examples of the present invention is carried out at room temperature. Room temperature means a temperature of 20°C ± 5°C.

### Example 1

0.7 g of lumateperone and 2.3 g of PLGA (lactide : glycolide = 50:50, carboxyl-terminated, molecular weight: 40,000 Daltons) were dissolved in 10 mL of dichloromethane, and the resulting solution was added into 300 mL of 0.5% PVA aqueous solution and sheared using a shearing machine at 1,000 rpm to form a primary emulsion, which was then volatilized under stirring at 200 rpm for 4 h, and filtered. The resulting microspheres were washed with distilled water for three times, and lyophilized to obtain lumateperone long-acting sustained-release microspheres. The particle size (D50) of the microspheres was measured as 57 µm by a laser particle size analyzer.

### Example 2

0.7 g of lumateperone and 2.3 g of PLGA (lactide : glycolide = 75:25, ester-terminated, molecular weight: 70,000 Daltons) were dissolved in 10 mL of dichloromethane, and the resulting solution was added into 300 mL of 0.5% PVA aqueous solution and sheared using a shearing machine at 1,000 rpm to form a primary emulsion, which was then volatilized under stirring at 200 rpm for 4 h, and filtered. The resulting microspheres were washed with distilled water for three times, and lyophilized to obtain lumateperone long-acting sustained-release microspheres. The particle size (D50) of the microspheres was measured as 61 µm by a laser particle size analyzer.

### Example 3

0.7 g of lumateperone and 2.3 g of PLA (molecular weight: 25,000 Daltons) were dissolved in 10 mL of dichloromethane, and the resulting solution was added into 300 mL of 0.5% PVA aqueous solution and sheared using a shearing machine at 1,000 rpm to form a primary emulsion, which was then volatilized under stirring at 200 rpm for 4 h, and filtered. The resulting microspheres were washed with distilled water for three times, and lyophilized to obtain lumateperone long-acting sustained-release microspheres. The particle size (D50) of the microspheres was measured as 59 µm by a laser particle size analyzer.

### Example 4

7 g of lumateperone and 23 g of PLA (molecular weight: 25,000 Dalton) were pulverized, and mixed uniformly for later use.

A Thermo Fisher Pharma 11 twin-screw hot melt extruder was started, and the parameters were set as follows: feeding temperature: room temperature, conveying temperature: 35-40°C, kneading temperature: 80-100°C, degassing temperature: 100°C, extrusion temperature: 80°C, die temperature: 60°C, pressure: 60 bar, extrusion speed: 100 rpm, torque: 7-8 N.cm.
Drawing into filaments and cutting into pellets: after extrusion, the resulting product was pulled and drawn into short rods of 3 × 30 mm;
Irradiation: the short rods were sterilized by irradiation at 18 kGy to obtain lumateperone subcutaneous implants.

### Example 5

0.7 g of lumateperone, 1.725 g of PLA (molecular weight: 25,000 Daltons), and 0.575 g of PLGA (lactide : glycolide = 75:25, ester group-terminated, molecular weight: 70,000 Daltons) were sequentially added into 10 ml of dichloromethane for dissolution, and the resulting solution was added into 300 mL of 0.5% PVA aqueous solution and sheared using a shearing machine at 1,000 rpm to form a primary emulsion, which was then volatilized under stirring at 200 rpm for 4 h, and filtered. The resulting microspheres were washed with distilled water for three times, and lyophilized to obtain lumateperone long-acting sustained-release microspheres.

### Test Example 1

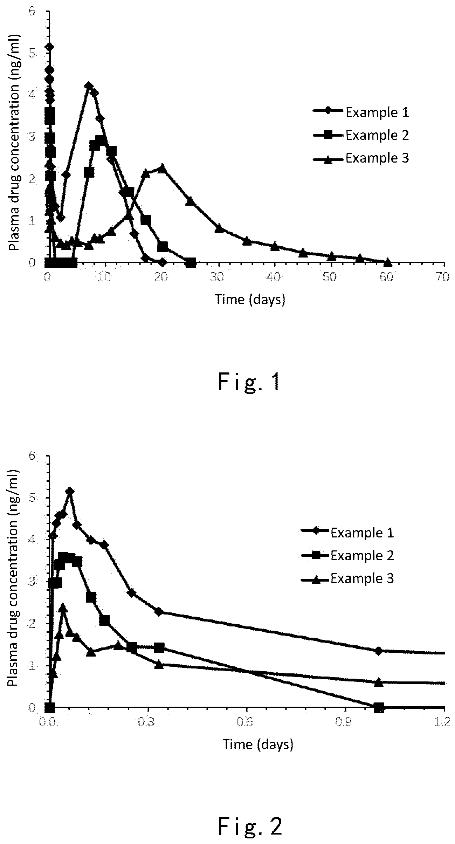

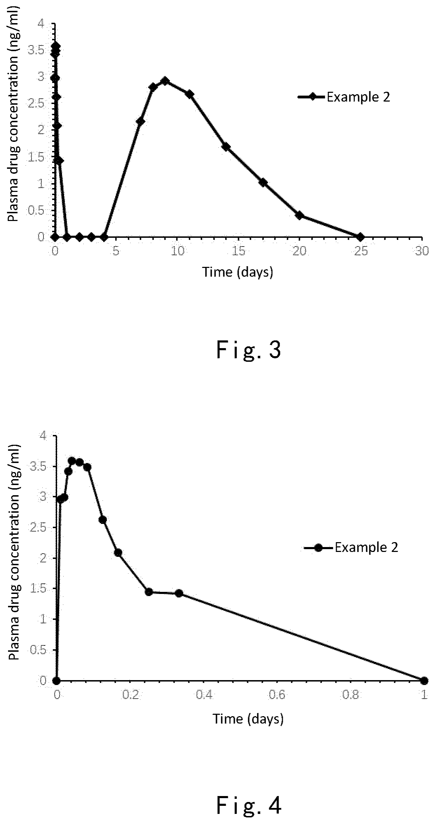

SD rats (n = 4, female) were intramuscularly injected with a single dose of 10.5 mg/kg of the microspheres from Examples 1-3 and 5, or subcutaneously implanted with 10.5 mg/kg of the subcutaneous implant from Example 4. The day of administration was recorded as 0 d. Blood samples were collected from the SD rats before administration (0 h) and at 15 min, 30 min, 45 min, 1 h, 1.5 h, 2 h, 3 h, 5 h (or 4 h or 6 h), 8 h, 1 d, 2 d, 3 d, 4 d (or 5 d), 7 d, 8 d, 9 d, 11 d, 14 d (or 13 d or 15 d), 17 d, 20 d, 25 d, 30 d, 35 d and 40 d after administration, respectively. Thereafter, according to the test results, blood was sampled every 5 d until complete release was achieved. 0.25 mL blood was taken from the tail vein, temporarily stored in wet ice, and centrifuged at 1,500 g for 10 min (4 °C) within 1 h to separate the plasma. The collected plasma was stored at -80°C in a refrigerator for subsequent analysis. The plasma drug concentration was measured post-administration, and the pharmacokinetic parameters were calculated by fitting the concentration-time curve. The results are shown in FIGs. 1-7.

From FIGs. 1-4, it can be observed that the microspheres of Example 1 exhibited an overly high initial burst release, while those of Example 2 showed a lag time of 4-5 days. In contrast, the microspheres of Example 3 demonstrated a significantly longer release duration, a notably reduced burst release and no lag period, indicating their suitability as a long-acting sustained-release formulation.

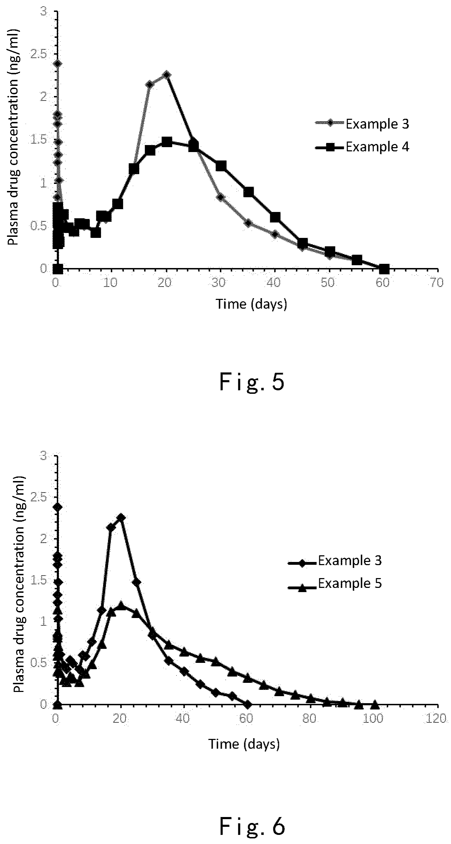

FIG. 5 reveals no significant difference in the release duration between the microspheres of Example 3 and the subcutaneous implants of Example 4. However, the release profile of the subcutaneous implant in Example 4 was smoother.

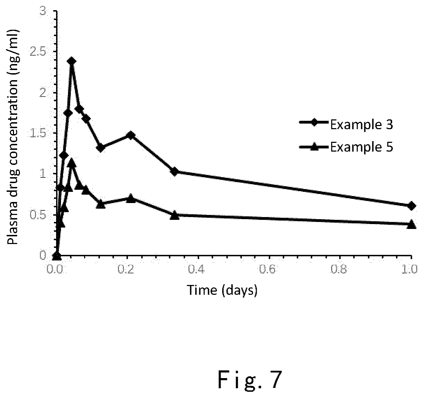

FIGs. 6 and 7 demonstrate that the microspheres of Example 5 had a longer release duration compared to those of Example 3.

While the methods and products of the present application have been described through preferred examples, it will be apparent to those skilled in the art that variations or suitable modifications and combinations can be made to the methods and products described herein to achieve the technique of the present application without departing from the content, spirit, and scope of the application. In particular, it should be pointed out that all such replacements and modifications would be obvious to those skilled in the art and be deemed to be included within the spirit, scope, and content of this application.

## Claims

1. A lumateperone long-acting sustained-release preparation composition, **characterized by** comprising lumateperone or a salt thereof and a polymer material, wherein the mass ratio of lumateperone to the polymer material is 0.5:9.5 to 5:5, the polymer material comprises a main material, polylactide, and optionally a release regulator, the mass of the release regulator is 0% to 95% of the mass of the main material polylactide, and the release regulator is selected from one of or a mixture of more than one of polylactide or poly(lactide-co-glycolide).

2. The lumateperone long-acting sustained-release preparation composition according to claim 1, **characterized in that** the mass ratio of lumateperone to the polymer material is 1:9 to 4:6; preferably, the mass ratio of lumateperone to the polymer material is 1.5:8.5 to 3:7.

3. The lumateperone long-acting sustained-release preparation composition according to claim 1 or 2, **characterized in that** the main material polylactide has a molecular weight of 5,000 to 100,000 Daltons, preferably 10,000 to 80,000 Daltons, more preferably 10,000 to 50,000 Daltons.

4. The lumateperone long-acting sustained-release preparation composition according to any one of claims 1 to 3, **characterized in that** the mass of the release regulator is 0% to 50%; preferably 0% to 40%; more preferably 0% to 30%, of that of the main material polylactide.

5. The lumateperone long-acting sustained-release preparation composition according to any one of claims 1 to 4, **characterized in that** the molar ratio of lactide to glycolide in the poly(lactide-co-glycolide) in the release regulator is 50:50 to 95:5, and the poly(lactide-co-glycolide) has a molecular weight of 5,000 to 100,000 Daltons; preferably, the molar ratio of lactide to glycolide is 50:50 to 85:15, and the poly(lactide-co-glycolide) has a molecular weight of 5,000 to 90,000 Daltons; more preferably, the molar ratio of lactide to glycolide is 50:50 to 75:25, and the poly(lactide-co-glycolide) has a molecular weight of 10,000 to 80,000 Daltons;
the polylactide in the release regulator has a molecular weight of 5,000 to 100,000 Daltons, preferably 5,000 to 80,000 Daltons, and more preferably 5,000 to 50,000 Daltons.

6. The lumateperone long-acting sustained-release preparation composition according to any one of claims 1 to 5, **characterized in that** the release regulator is poly(lactide-co-glycolide) with a molar ratio of lactide to glycolide of 50:50 to 85:15 and a molecular weight of 5,000 to 90,000 Daltons; preferably, the release regulator is poly(lactide-co-glycolide) with a molar ratio of lactide to glycolide of 50:50 to 75:25 and a molecular weight of 10,000 to 80,000 Daltons; preferably, the release regulator is poly(lactide-co-glycolide) with a molar ratio of lactide to glycolide of 75:25 and a molecular weight of 10,000 to 80,000 Daltons.

7. The lumateperone long-acting sustained-release preparation composition according to any one of claims 1 to 6, **characterized in that** the polymer material is polylactide; or the polymer material is a mixture of polylactide and poly(lactide-co-glycolide), and the mass of the poly(lactide-co-glycolide) is 20% to 50% of that of the polylactide.

8. The lumateperone long-acting sustained-release preparation composition according to any one of claims 1 to 7, **characterized in that** the salt of lumateperone is selected from a group consisting of erucate, oleate, α-linolenate, palmitate, heptadecanoicate, undecylenate, hexadecanedionate, laurate, stearate, adipate and benzoate of lumateperone.

9. The lumateperone long-acting sustained-release preparation composition according to any one of claims 1 to 8, selected from microspheres or subcutaneous implants.

10. A method for preparing the lumateperone long-acting sustained-release microspheres according to claim 9, comprising the steps of:
dissolving the polymer material and the lumateperone or a salt thereof in a solvent, sterilizing by filtration to obtain an oil phase, and controlling the temperature at 2-35°C; preparing a 0.2% to 5% PVA aqueous solution, sterilizing by filtration to obtain an aqueous phase, and controlling the temperature at 2-35°C; adding the oil phase into the aqueous phase in a shearing machine to shear to form a primary emulsion, then volatilizing under stirring, filtering, washing microspheres with distilled water, and lyophilizing to obtain the lumateperone long-acting sustained-release microspheres.

11. A method for preparing the lumateperone long-acting sustained-release subcutaneous implants according to claim 9, comprising the steps of:
mixing the polymer material and the lumateperone or a salt thereof uniformly, extruding, then drawing into filaments and cutting into pellets, and sterilizing by irradiation to obtain the lumateperone long-acting sustained-release subcutaneous implants;
preferably, the extrusion is perfomed with a twin-screw extruder;
preferably, the extrusion parameters of the twin-screw extruder are as follows: feeding temperature: 15-30°C, conveying temperature: 35-40°C, kneading temperature: 60-100°C, degassing temperature: 80-100°C, extrusion temperature: 50-80°C, die temperature: 50-60°C, extrusion speed: 80-120 rpm, torque: 7-8 N.cm;
more preferably, the extrusion parameters of the twin-screw extruder are as follows: feeding temperature: room temperature, conveying temperature: 35-40°C, kneading temperature: 80-100°C, degassing temperature: 100°C, extrusion temperature: 80°C, die temperature: 60°C, extrusion speed: 100 rpm, torque: 7-8 N.cm.
